# EUROPEAN PATENT SPECIFICATION

(11) **EP 2 362 761 B1**
(45) Date of publication and mention of the grant of the patent: **12.07.2017**
(21) Application number: 09815041.0
(22) Date of filing: 15.09.2009
(51) Int. Cl.: A61F 2/32, A61F 2/36, A61F 2/34, A61F 2/30, A61B 17/15, A61B 17/16, A61F 2/40, A61F 2/46, A61B 17/17

(54) **APPARATUS FOR ADDRESSING FEMORAL ACETABULAR IMPINGEMENT**
GERÄT ZUR BEHANDLUNG VON FEMOROACETABULÄREM IMPINGEMENT
DISPOSITIF POUR TRAITER UN PINCEMENT FÉMORO-ACÉTABULAIRE

(30) Priority: 18.09.2008 US 98105 P
(43) Date of publication of application: 07.09.2011
(73) Proprietor: Smith & Nephew, Inc., Memphis, TN 38116 (US)
(72) Inventor: MASONIS, John, L., Charlotte North Carolina 28204 (US); COOPER, Michael, Nesbit Mississippi 38651 (US); KELMAN, David, C., Collierville Tennessee 38017 (US)
(74) Representative: Smith & Nephew
(86) International application number: PCT/US2009/056890
(87) International publication number: WO 2010/033473

(56) References cited:
- US-A- 5 376 125
- US-A- 5 913 899
- US-A- 5 913 899
- US-A- 6 162 257
- US-A- 6 162 257
- US-A1- 2005 182 493
- US-A1- 2005 182 493
- US-B1- 6 416 553

## Description

### CROSS-REFERENCE TO RELATED APPLICATIONS

This application claims priority to U.S. Provisional Application No. 61/098,105, filed on September 18, 2008.

### FIELD OF ART

The present invention relates generally to hip implants and more particularly to an early intervention hip device for addressing femoral acetabular impingement indications.

### RELATED ART

The features of the preamble of claim 1 are known from US-A-6162257. Femoral acetabular impingement (FAI) is a condition where extra bone on the femoral neck hits the rim of the acetabulum, wherein surrounding cartilage and labrum can be damaged. These tissues are the cushion between the ball and socket, and when damaged, can cause pain and further degeneration of the hip joint. Degeneration begins with labral and chondral rim lesions from impingement. If left untreated, pain and arthritis continues and a hip resurfacing procedure or total hip replacement (THR) is needed.

Treatment of FAI is necessary when there is chondral loss at the anterior acetabular rim and/or an osseous prominence on the femoral neck. The latter is commonly referred to as a "cam" deformity, which is clearly illustrated in figures 2a-2b. Alternatively, a prominence on the acetabular rim is commonly referred to as a "pincer-type" FAI deformity. The pincer-type deformity is clearly illustrated in figures 3a-3b. FAI may be associated with both cam and pincer-type deformities (shown in figures 4a-4b) and may lead to a combination of cam and pincer-type impingement.

There are currently few options to treat FAI without completing a total hip arthroplasty (THA) on the affected joint. Non-operative management of FAI is possible; however, it generally involves a change in lifestyle from an active to a less active lifestyle. Moreover, a commitment to maintaining hip strength is necessary. Non-operative management may not change the underlying abnormal hip biomechanics of FAI and may not fully alleviate the patient pain.

Operative management of FAI can be addressed via hip arthroscopy or open surgery. In hip arthroscopy, the hip is fully distracted and an arthroscope (with irrigation fluid) is used to make an assessment of the hip joint and treat damage that is found through two to four 1 cm incisions. While arthroscopy is useful for diagnosis and treating labral tears, there is currently no clinical data on treating damaged cartilage areas affected by FAI. A hip arthroscopy involving labral debridement (no repair) and no bony decompression usually takes less than one hour. A hip arthroscopy involving labral/cartilage repair and FAI decompression may take between two and four hours, depending on the amount of work performed. Care must be taken to avoid damage to the hip's blood supply (the retinacular vessels) during the osteoplasty procedure.

The open surgical hip dislocation involves an incision (approximately 6 to 10 inches), an osteotomy or bone cutting of the trochanter, and a complete dislocation of the femoral head from the acetabulum exposing all parts of the joint. This exposure allows treatment of labral tears and abnormal contact between the ball and socket while protecting the blood supply to the hip. The open approach can typically be done in a few hours. The open approach is not generally recommended in older patients or in patients with significant hip degeneration. While the open surgical approach provides good visualization, it is invasive, because soft tissues are compromised during complete dislocation leading to longer recovery time and higher levels of post-operative pain. The present invention allows a minimally-invasive direct anterior technique using partial distraction, rather than complete dislocation, and therefore is better-suited for a larger patient population.

With conventional open surgical approaches, if the hip has not degenerated too far, FAI is treated by surgically dislocating the hip, trimming the excessive portion of the front of the socket, and then reshaping the junction between the head and neck of the femur to give the joint more clearance and reduce cam impingement. The effect of this reshaping of the joint can easily be seen at the time of surgery when the joint is opened. There has been very little written about this problem thus far, but it has been shown to be very helpful if performed on the right hip at the right time.

There is a large patient population affected by FAI, wherein trauma to the cartilage is not fixable using convention arthroscopic methods, but is also not excessive enough to justify a hip resurfacing procedure or a total hip arthroplasty (THA). In some practices, up to 70% of young and active patients fall into this patient population. Currently, there is no known arthroscopic or orthopedic solution for this large patient population, and therefore, there is a need to provide a minimally-invasive early intervention FAI solution for said large patient population. The present invention serves to provide said large patient population affected by FAI with an early intervention alternative to conventional hip resurfacing and THA where traditional arthroscopic procedures are not applicable, not recommended, or not effective.

### SUMMARY

The aforementioned needs are satisfied by an apparatus according to claim 1.

According to some aspects, there is provided early intervention acetabular implant which may be used to treat femoral acetabular impingement.

According to other aspects, there is provided a method for manufacturing said early intervention acetabular implant.

According to yet another aspect, instrumentation for installing said early intervention acetabular implant is provided.

According to other aspects, there are provided methods for surgically preparing a bony site for receiving one or more early intervention acetabular implants, said methods including one of a direct anterior approach and a trochanteric flip/surgical?disclocation.

According to yet other aspects there are provided less-invasive methods for viewing an acetabulum, including the step of visualizing a joint gap space with a dry arthroscope.

According to yet even other aspects, there are provided methods for surgically implanting an early intervention acetabular implant.

According to other aspects, there are provided methods of performing studies of femoral acetabular impingement.

According to yet another aspect, there are provided methods of evaluating and optimizing early intervention acetabular implants designed to reduce femoral acetabular impingement symptoms.

The present invention relates to an apparatus for treating a rim deformity of an acetabulum. The apparatus comprises an implant a substantially having a substantially L-shaped profile in the anterior posterior coronal view. The implant has a first flange and a second flange. The first flange is configured to extend into the acetabulum and the second flange is configured substantially perpendicular to the first flange to extend over the rim of the acetabulum. A fixation element is configured to extend through the second flange and fix the implant to chondral bone. The first flange comprises a bearing surface for a femoral head to articulate against.

In an embodiment, the second flange has anteriorly and posteriorly extending flanges when viewed laterally.

In yet another embodiment, the width of the first and second flanges is less than or equal to 2 cm and the thickness of the first and second flanges is between 2 and 6 mm thick.

A further embodiment includes a screw as the fixation element. In an embodiment, the bearing surface is for natural femoral head cartilage to articulate against.

The present description also discloses a method for treating femoral acetabular impingement in an acetabulum. A step surgically exposes a hip joint through a direct anterior approach. A step distracts the hip joint by separating a femur from the acetabulum. Another step removes a portion of the rim of the acetabulum by removing a first portion extending into the acetabular socket and a second portion extending generally perpendicular to the first portion over the rim of the acetabulum. Additionally, the method requires positioning an implant within the removed first and second portion of the rim of the acetabulum. A step releases the hip joint distraction. Another step secures the implant to chondral bone in the acetabulum with a fixation element.

Another method includes the implant comprising an implant generally having an L-shaped profile in the anterior posterior coronal view. The implant has a first flange and a second flange. The first flange is configured to extend into the acetabulum and the second flange is configured generally perpendicular to the first flange to extend over the rim of the acetabulum. A fixation element is configured to extend through the second flange and fix the implant to chondral bone.

In one embodiment, the removing step further comprises the steps of fixing a cutting guide to the femur and rotating the femur relative to the acetabulum such that the cutting guide cuts the acetabulum.

Alternatively, the removing step may comprise burring the first portion and second portion from the acetabulum.

In an embodiment wherein the removing step comprises burring, the burr may be moved with the femur relative to the acetabulum.

In yet another aspect, the releasing step may be completed before the securing step.

Further areas of applicability of the present invention will become apparent from the detailed description provided hereinafter. It should be understood that the detailed description and specific examples, while indicating certain embodiment of the invention, are intended for purposes of illustration only and are not intended to limit the scope of the invention.

### BRIEF DESCRIPTION OF THE DRAWINGS

The accompanying drawings, which are incorporated in and form a part of the specification, illustrate the embodiments of the present invention and together with the written description serve to explain the principles, characteristics, and features of the invention. In the drawings:

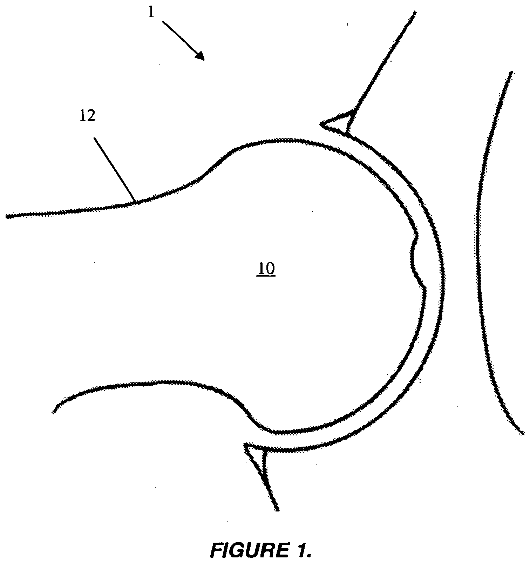

FIG. 1 is an anterior-posterior coronal view of a normal hip joint;

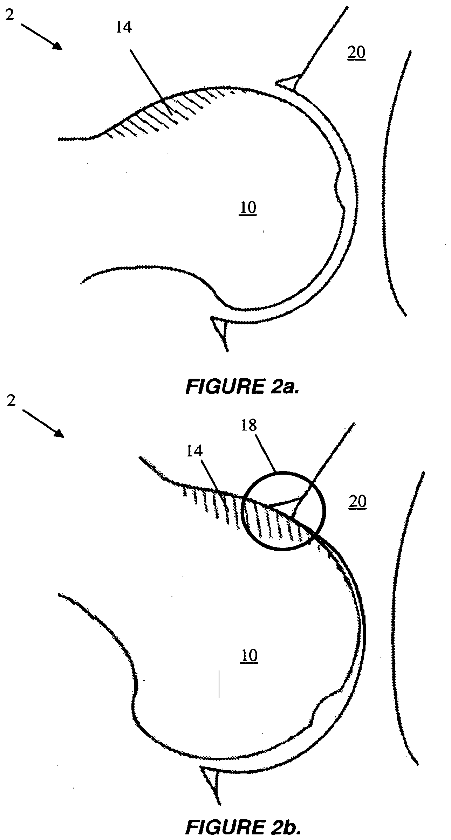

FIG. 2a is an anterior-posterior coronal view of an extended hip joint having a cam-type neck deformity and cam impingement zone;
FIG. 2b is an anterior-posterior coronal view of an impinging and abducted hip joint having a cam-type neck deformity;

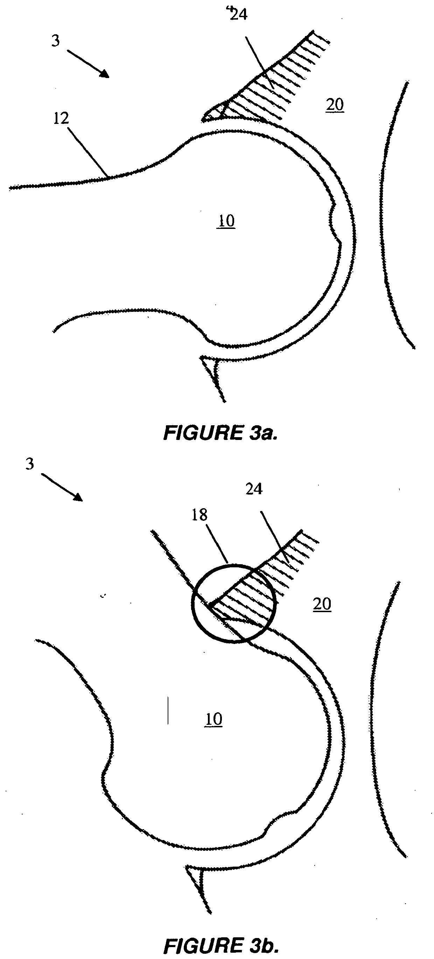

FIG. 3a is an anterior-posterior coronal view of an extended hip joint having a pincer-type rim deformity and pincer impingement zone;
FIG. 3b is an anterior-posterior coronal view of an impinging and abducted hip joint having a pincer-type rim deformity;

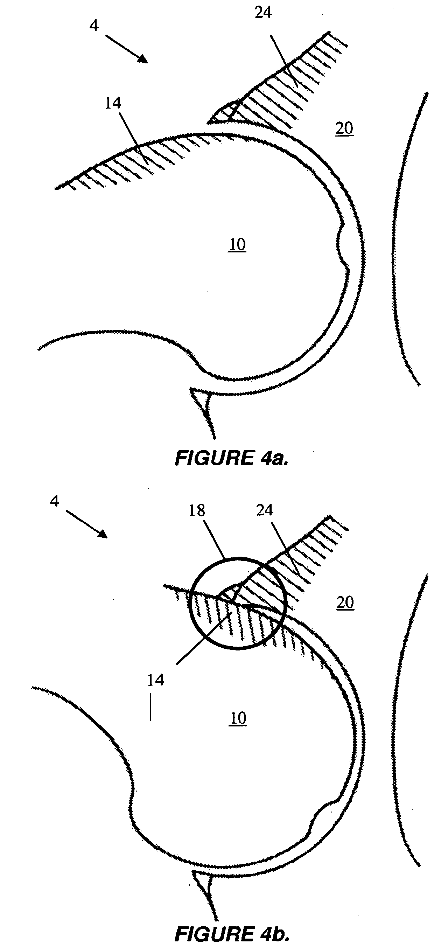

FIG. 4a is an anterior-posterior coronal view of an extended hip joint having both a cam-type neck deformity and a pincer-type rim deformity with associated impingement zones;
FIG. 4b is an anterior-posterior coronal view of an impinging and abducted hip joint having both a cam-type neck and pincer-type rim deformities;

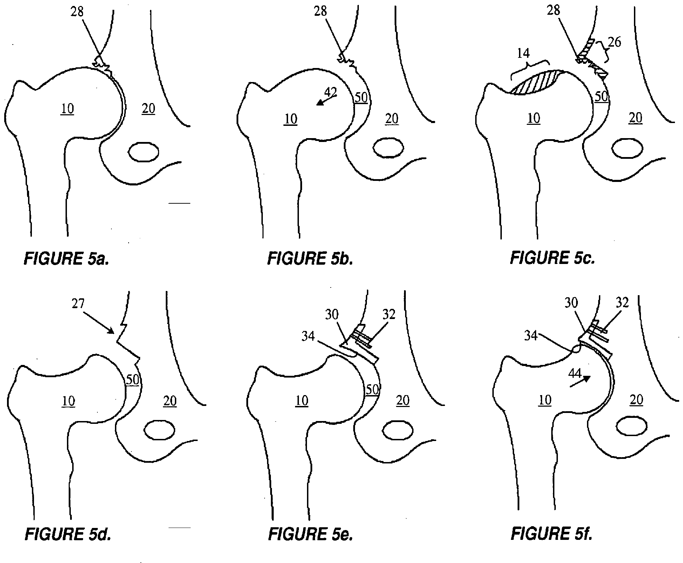

FIG. 5a is an anterior-posterior coronal view of a FAI-affected hip joint;
FIG. 5b illustrates the step of distracting the hip joint of FIG. 5a;
FIG. 5c illustrates areas of bone and cartilage of a hip joint to be removed;
FIG. 5d illustrates the hip joint of FIG. 5a-5c with removed bone and cartilage;
FIG. 5e illustrates the hip joint of FIG. 5a-5d with acetabular resurfacing implant according to the present invention installed;
FIG. 5f is an anterior-posterior coronal view of the hip joint shown in FIG. 5e in a retracted state.

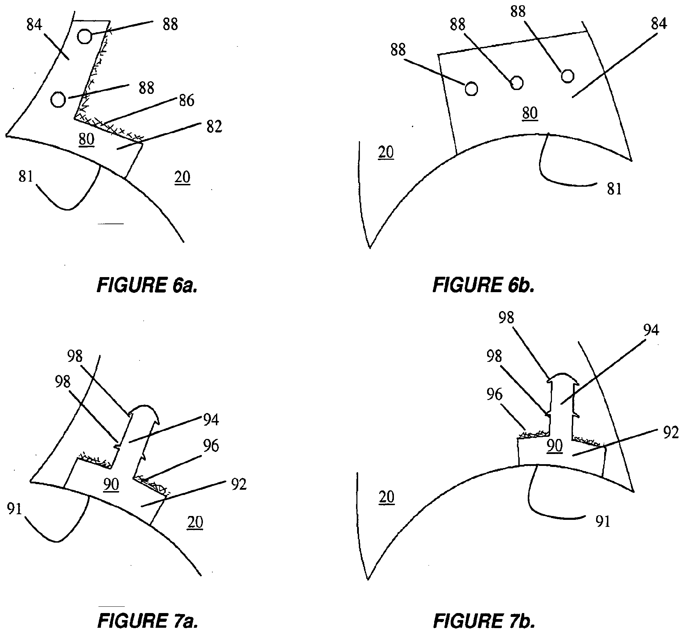

FIG. 6a is an anterior-posterior coronal view of a hip joint comprising an implant according to the present invention;
FIG. 6b is a lateral sagittal view of the hip joint shown in FIG. 6a;
FIG. 7a is an anterior-posterior coronal view of a hip joint comprising an implant not according to the present invention;
FIG. 7b is a lateral sagittal view of the hip joint shown in FIG. 7a;

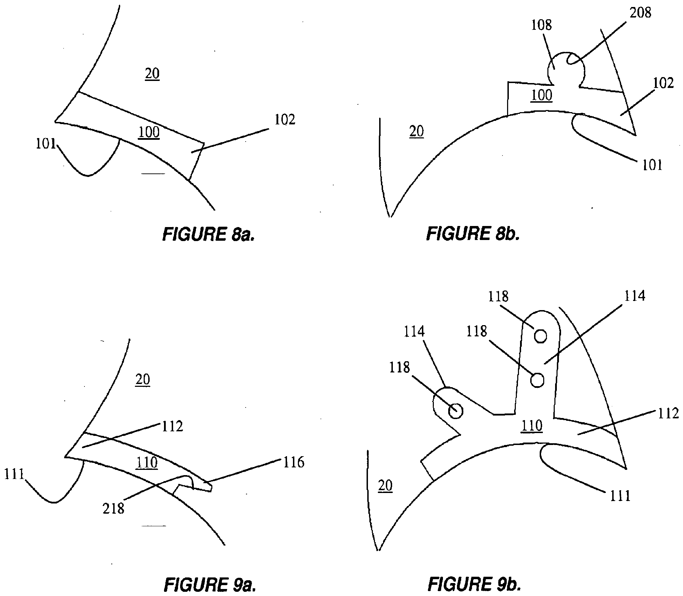

FIG. 8a is an anterior-posterior coronal view of a hip joint comprising an implant not according to the present invention;
FIG. 8b is a lateral sagittal view of the hip joint shown in FIG. 8a;
FIG. 9a is an anterior-posterior coronal view of a hip joint comprising an implant. not according to the present invention;
FIG. 9b is a lateral sagittal view of the hip joint shown in FIG. 9a;

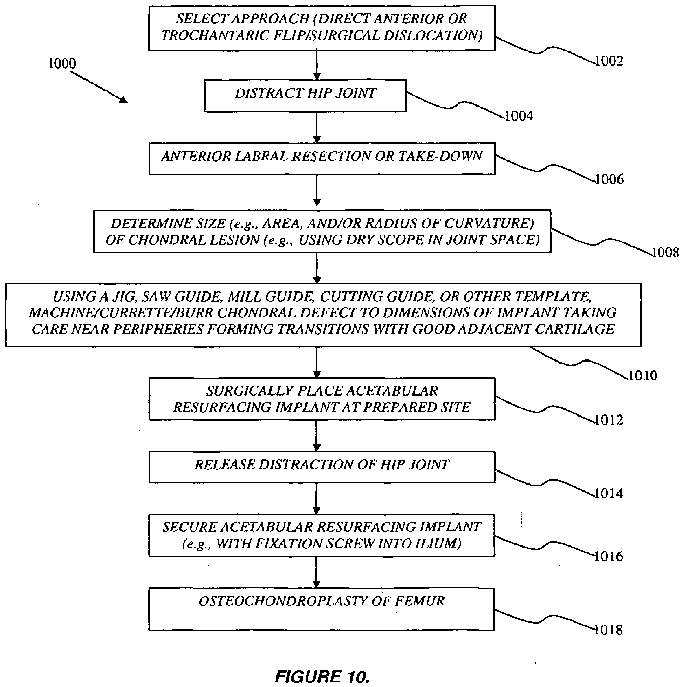

FIG. 10 is a schematic diagram illustrating method steps for installing the implants of the present invention;

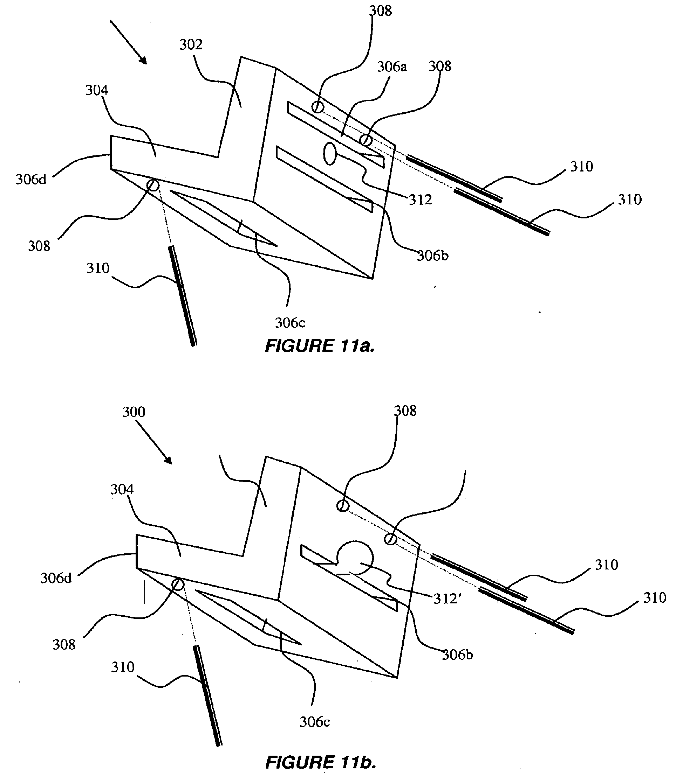

FIG. 11a illustrates a cutting jig ;
FIG. 11b illustrates a cutting jig , particularly for use with implants having *tongue-in-groove*-type locking features such as the one shown in FIGS. 8a-b;

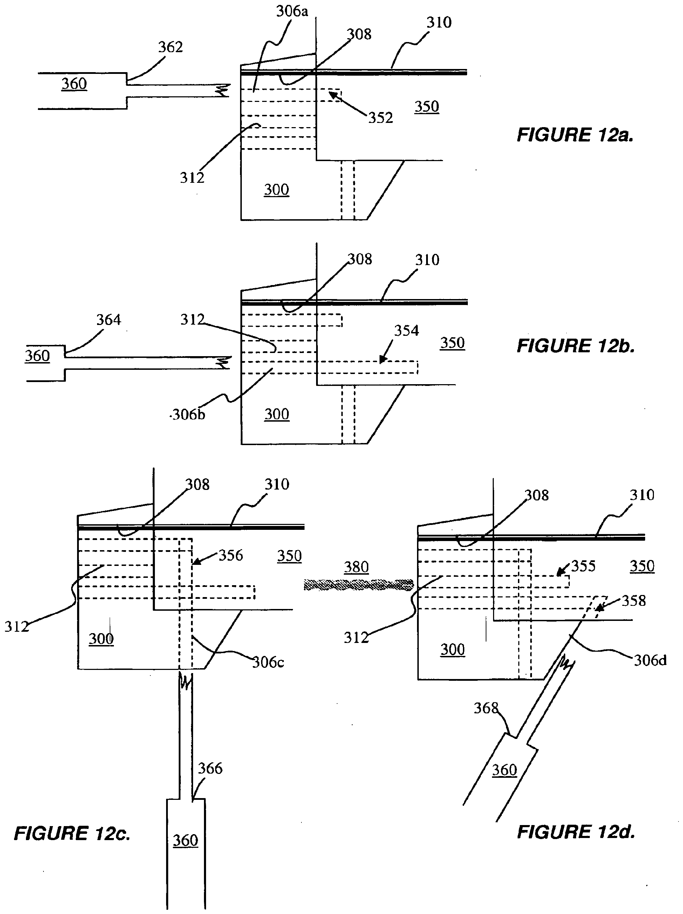

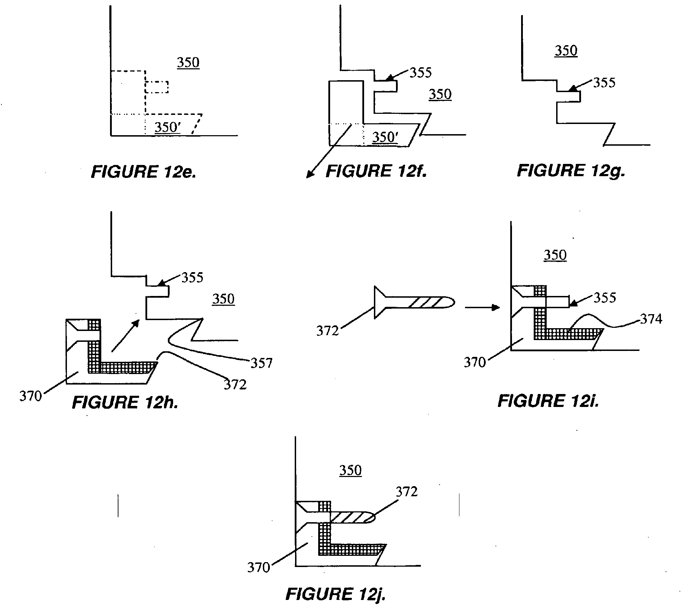

FIGS. 12a-12j illustrate method steps of using the cutting jig shown in FIG. 11a;

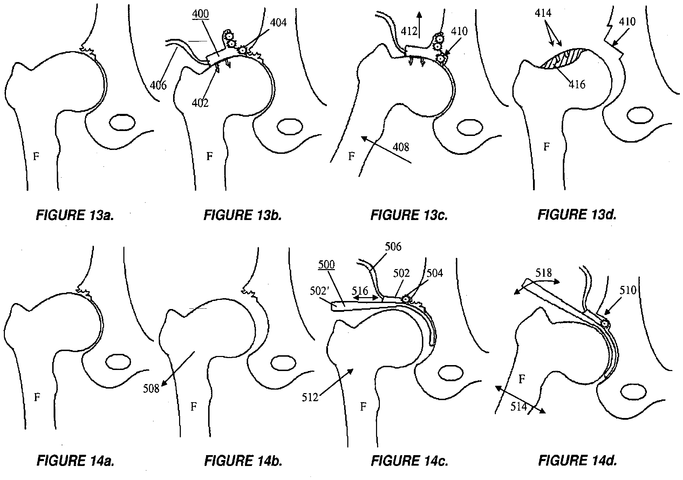

FIGS. 13a-d illustrate method steps of using rotating cutters to mill the defect area ;
FIGS.14a-d illustrate method steps of using rotating cutters to mill the defect area ;

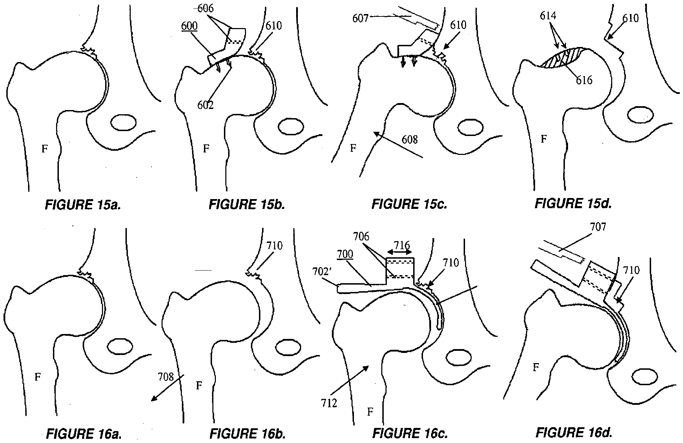

FIGS. 15a-d illustrate method steps of using a cutting block mounted to a proximal femur in order to resect the defect area ;
FIGS. 16a-d illustrate method steps of using a cutting block mounted to a spacer insertable in a joint gap space in order to resect the defect area;

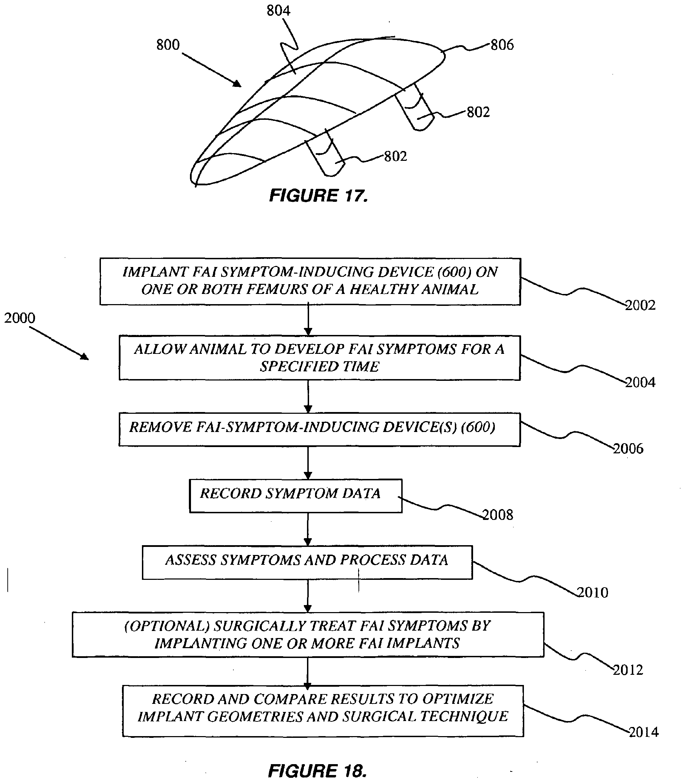

FIG. 17 depicts a femoral acetabular impingement symptom-inducing device for use in animal testing and FAI research; and,
FIG. 18 is a schematic diagram illustrating method steps for using the device shown in FIG. 17.

### DETAILED DESCRIPTION OF THE EMBODIMENTS

The following description of the preferred embodiment(s) is merely exemplary in nature and is in no way intended to limit the invention, its application, or uses.

The invention may provide, in part, a partial acetabular resurfacing construct which may be utilized for hemi-acetabular articulation with natural femoral head cartilage. The construct is configured for preventing further degeneration of a healthy femoral head and its surrounding tissues, and may be considered a novel solution for indications between arthroscopic intervention and conventional hip resurfacing or THA. It is to be noted, however, that the present invention may also be used to articulate with femoral heads, however; such uses are not preferable.

The acetabular resurfacing construct generally comprises a bearing surface, and a means for bio-fixation which preferably does not utilize cement. The means for bio-fixation may comprise, for instance, one or more of a porous construct and screws with locking heads. The resurfacing construct is generally revisable to a conventional hip-resurfacing or total hip arthroplasty procedure, and therefore is preferably low-in profile. In other words, the resection profiles for constructs of the present invention are preferably within the resection profiles for resurfacing and conventional acetabular shells. The acetabular resurfacing constructs of the present invention may come in various shapes, forms, and materials; however, the articulating bearing surface should be optimized for a cartilage bearing couple (e.g., may be oxidized zirconium, cobalt chromium, ceramic, polymer, or a bioscaffold). It is anticipated that the bearing surface may comprise different radii to accommodate differently-sized femoral heads. For instance, a set of implants according to some embodiments of the present invention may comprise multiple curvatures with incremental medial-lateral or anterior-posterior sizes within each curvature to accommodate different defect areas.

It is preferred that the transition surfaces between implants and surrounding cartilage and tissues are smooth and continuous so as to form a smooth homogeneous bearing surface.

The present invention is limited to acetabulum.

FIG. 1 illustrates a normal hip joint (1) comprising a proximal femur (10) and acetabulum (20). The femur (10) has a normal femoral neck (12) and the rim of the acetabulum (20) is normal. The labrum, acetabular rim, adjacent tissues, and chondral surfaces are generally normal in this femoral (10) and acetabular (20) configuration.

FIG. 2a illustrates an FAI-affected hip joint (2) comprising a proximal femur (10) and acetabulum (20). The hip joint (2) is of the "cam" deformity type which causes cam impingement. The femur (10) has a femoral neck protuberance (14), whereas the rim geometry of the acetabulum (20) is generally normal. Impingement zone (18) is shown in figure 2b. The labrum, acetabular rim, adjacent tissues, femoral neck, and chondral surfaces within impingement zone (18) are generally compromised in this femoral (10) and acetabular (20) configuration, because the femoral neck protuberance (14) impinges with the rim of the acetabulum (20) during certain leg motions.

FIG. 3a illustrates an FAI-affected hip joint (3) comprising a proximal femur (10) and acetabulum (20). The hip joint (3) is of the pincer-deformity type. The femur (10) has a normally shaped femoral neck (12), whereas the rim geometry of the acetabulum (20) has a rim protuberance (24). Impingement zone (18) is shown in figure 3b. The labrum, acetabular rim, adjacent tissues, femoral neck, and chondral surfaces within impingement zone (18) are generally compromised in this femoral (10) and acetabular (20) configuration, because the femoral neck (12) impinges with the enlarged rim of the acetabulum (20) during certain leg motions.

FIG. 4a illustrates an FAI-affected hip joint (4) comprising a proximal femur (10) and acetabulum (20). The hip joint (4) is of both the cam and pincer-deformity type. The femur (10) has a femoral neck protuberance (14), and the rim geometry of the acetabulum (20) has a rim protuberance (24). Impingement zone (18) is shown in figure 4b. The labrum, acetabular rim, adjacent tissues, femoral neck, and chondral surfaces within impingement zone (18) are generally compromised in this femoral (10) and acetabular (20) configuration, because the femoral neck protuberance (14) impinges with the enlarged rim of the acetabulum (20) during certain leg motions.

FIGS. 5a-5e illustrate a method of implanting a device according to some embodiments of the present invention.

FIG. 5a illustrates an anterior-posterior coronal view of a FAI-affected hip joint. Hip joint includes a proximal femur (10) having an enlargement or shape deformity and acetabulum (20). Over time, the proximal femur (10) impinges with the acetabulum (20) creating a compromised zone (28) (e.g., labrum tear, chondral defect, delaminated cartilage) in or around the acetabulum (20) adjacent to the impingement site (18).

FIG. 5b illustrates the step of distracting the FAI-affected hip joint of FIG. 5a. In order to create a working space and visualize the acetabular defect, a direct anterior approach may be utilized to preserve tissues. The hip joint is then distracted (42) enough to form an operable gap space (50) between the femur (10) and acetabulum (20). Extent of damage to the labrum or surrounding cartilage can be assessed by inserting a dry arthroscope (not illustrated) within said formed gap space (50). Sizing and measuring of the chondral defect(s) may further be assessed using said dry arthroscope. Curvature of the bearing surface of the acetabulum (20) may be done at this point using various inserts, spoons, and/or visually with said dry arthroscope. Conventional templating on X-ray or other radiological methods may be utilized to determine radius of curvature.

FIG. 5c illustrates the method step of determining areas of bone and cartilage of a hip joint to be removed. A portion (s) (14) of the femoral neck may be removed to provide more clearance between the femur and rim of the acetabulum (20). A predetermined portion or portions (26) of acetabular cartilage, tissues, and/or bone adjacent the compromised zone (28) may be removed. The redetermined portion (26) generally corresponds to the size, shape, and thickness of the implant to be inserted.

FIG. 5d illustrates the hip joint of FIG. 5a-5c with portions (14, 26) of bone and cartilage removed from areas on or adjacent to the acetabulum (20). Removing portions (14, 26) from the acetabular area forms an implant space (27). Implant space (27) is generally configured and sized according to the implant to be used.

The implant space may be slightly smaller in size than the implant so that the implant may rest proud relative to bone when the implant is implanted. The removal of bone may be limited to particular depths so that a revision to a hip resurfacing application may be accomplished. Such a revision may only be accomplished if the removal of bone is relatively minimal. Preferably, the amount of bone removal would be less than 5 mm thick, and more preferable less than 3 mm thick.

FIG. 5e illustrates the hip joint of FIG. 5a-5d with a partial acetabular resurfacing implant (30) installed. The implant (30) comprises a bearing surface (34) configured to match the general concavity of the surrounding acetabulum (20) and is generally composed of a material that articulates well against natural femoral head cartilage. Implant (30) further comprises a means for bio-fixation through fixation elements (32), which may include, for example and without limitation, porous constructs, one or more screws or pins, spikes, tacks, snap connectors, posts, barbs, holes, channels, tongue-in-groove-type locking mechanisms, mounting flanges, protruding lips, and/or combinations thereof.

The implant (30) may be generally L-shaped in the anterior posterior coronal view in order to provide proper strength to the implant while minimizing bone removal. The flanges of the implant (30) may then extend over the defect zone without extending deep into the underlying bone. In most embodiments, the flanges will not exceed 2 cm, while the thickness may be within the range of 2 to 6 mm. For example, in one embodiment, the flanges of the L-shaped implant (30) may be approximately 1 cm in length. The thickness of the flanges, though, may be 3 mm thick. Thus, if the implant (30) is to sit flush with the cartilage of the acetabulum, the total thickness of the bone removed from the acetabulum would be less than 3 mm thick. In such an embodiment, a resurfacing application where 3 mm thickness of tissue and bone is to be removed from the acetabulum in order to resurface it, the implant (30) would not cause any gaps between the shell and bone.

FIG. 5f is an anterior-posterior coronal view of the hip joint shown in FIG. 5e in a retracted state. Retraction (44) closes the gap space (50) such that natural cartilage on the head of the femur (10) can articulate against both the bearing surface (34) of the implant (30), and also the remaining natural cartilage on the acetabulum (20) that was not compromised by FAI or other conditions. Retraction (44) may precede the step of removing portion (14) of the femoral neck, in order to protect the bearing surface (34) of the implant (30) from accidental burring.

FIGS. 6a-6b illustrate an implant (80) according to some embodiments of the present invention. FIG. 6a is an anterior-posterior view of an acetabular rim having a repaired chondral defect associated with FAI. An "L"-shaped implant (80) having a lower portion (82) and side portion (84) is secured to the acetabular rim using a means for bio-fixation. The side portion (84) may comprise additional flanges to form a 3-plane corner. The lower portion (82) comprises a bearing surface (81) which is preferably shaped according to the size and curvature of the surrounding acetabular socket geometry. The bearing surface may, for instance comprise oxidized zirconium, ceramic, cobalt chromium, or other suitable biocompatible articulations. The means for bio-fixation may include one or more straight or oblique 3.5mm locking screw holes (88) configured for use with locking screws. The means for bio-fixation may also include one or more ingrowth-enhancement structures (86) for example, a porous construct, sintered beads, titanium foam, and/or combinations thereof. FIG. 6b is a lateral view of the installed implant shown in FIG. 6a. While the view of Figure 6b shows a single flange extending in a generally rectangular shape, other shapes, such as multiple flanges extending anteriorly and posteriorly on the lateral view, may be employed to provide better fixation while minimizing bone removal. In such an embodiment, the anterior lateral coronal view of the implant is still gernally L-shaped, but the lateral view shows flanges diverging.

FIGS. 7a-7b illustrate an implant (90) not according to the present invention. FIG. 7a is an anterior-posterior view of an acetabular rim having a repaired chondral defect associated with FAI. A button-shaped implant (90) having a lower portion (92) and shaft portion (94) is secured to the affected acetabulum area using a means for bio-fixation. The means for bio-fixation may be located on one or both of the shaft portion (94) and the lower portion (92). The lower portion (92) comprises a bearing surface (91) which is preferably concaved and shaped according to the size and curvature of the surrounding acetabular socket geometry. The bearing surface may, for instance, comprise oxidized zirconium, ceramics, diamond or diamond-like carbon, cermets, cobalt chromium, nitrides, or other suitable biocompatible articulation materials. The means for bio-fixation may include one or more of barbs, ribs, or protuberances (98) alone or in combination with ingrowth-enhancement structures (96) (e.g., porous constructs, sintered beads, titanium foam, and/or combinations thereof). While not shown in FIG. 7a, the shaft portion (94) may include an ingrowth-enhancement structure (96). Moreover, shaft portion (94) may take many forms including one or more posts, pegs, spikes, keels, and combinations thereof. Bottom portion (92) may come in a variety of thicknesses, shapes, and sizes to accommodate different chondral defects for different patients. The edges of the bearing surface (91) may be rounded to eliminate a sharp interface between cartilage and the FAI implant. FIG. 7b is a lateral view of the installed implant shown in FIG. 7a.

FIGS. 8a-8b illustrate an implant (100) not according to the present invention. FIG. 8a is an anterior-posterior view of an acetabular rim having a repaired chondral defect associated with FAI. An extruded implant (100) having a lower portion (102) is secured to the acetabular rim using a means for bio-fixation. The lower portion (102) comprises a bearing surface (101) which is preferably shaped according to the size and curvature of the surrounding acetabular socket geometry. The bearing surface is preferably optimized for a cartilage bearing couple and may, for instance and without limitation, comprise oxidized zirconium, ceramic, cobalt chromium, polyethylene, PEEK, bioscaffolds, other suitable biocompatible articulations, and combinations thereof. The means for bio-fixation may include a key-type tongue-in-groove locking mechanism (108). A keying broach instrument rasps out a keyway along the acetabular rim, so as to provide a channel (208) for locking mechanism (108) to slide into and frictionally engage. Locking mechanism (108) may come in many cross-sectional forms, including a circle, trapezoid (as shown in FIG. 8b), complex spline, or polygon. A circular cross-sectional form would allow a drill bit or pin to create the channel (208). Additional means for bio-fixation may be used in addition to the locking mechanism (108) in order to secure the implant (100) from sliding within the channel (208). Such additional means for bio-fixation may be any of the means for bio-fixation mentioned in this specification, including, but not limited to 3.5mm locking screws and/or ingrowth-enhancement structures (86,96). FIG. 8b is a lateral view of the installed implant shown in FIG. 8a.

FIGS. 9a-9b illustrate an implant (110) not according to the present invention. FIG. 9a is an anterior-posterior view of an acetabular rim having a repaired chondral defect associated with FAI. A flanged implant (110) is secured to the acetabular rim using a means for bio-fixation. The implant (110) comprises a lower portion (112) having a bearing surface (111) which is preferably shaped according to the size and curvature of the surrounding acetabular socket geometry. The bearing surface is preferably optimized for a cartilage bearing couple and may, for instance and without limitation, comprise oxidized zirconium, ceramic, cobalt chromium, polyethylene, PEEK, bioscaffolds, other suitable biocompatible articulations, and combinations thereof. The means for bio-fixation may include a a lip flange (116) locking mechanism. A keying broach instrument rasps out a keyway along the acetabular rim, so as to provide a channel (218) for the lip flange (116) locking mechanism to slide into for stability in at least one spatial degree-of-freedom. Lip flange (116) may come in many cross-sectional forms, including a trapezoidal (as shown in FIG. 9a). Sizes and shapes of lip flange (116) may optimized for stability and fixation and simplified to streamline instrumentation. For example, a circular cross-sectional form for lip flange (116) would allow a drill or pin to create the channel (218). Lip flange (116) may be interrupted and comprise a protruding spike, tack, or sharpened corrugated ridge. Additional means for bio-fixation may be used in addition to the lip flange (116) in order to secure the implant (110) from sliding out of the channel (218). Such additional means for bio-fixation may be any of the means for bio-fixation mentioned in this specification, including 3.5mm locking screws and/or one or more ingrowth-enhancement structures (86,96). FIG. 9b is a lateral view of the installed implant shown in FIG. 9a, and illustrates additional means for bio-fixation comprising at least one rim-mounting flange (114) and at least one or more straight or oblique 3.5mm locking screw holes (118) configured for use with locking screws.

FIG. 10 is a schematic diagram illustrating method steps for installing the implants of the present invention. First, an approach is selected (1002). For example, a minimally-invasive direct anterior approach may be advantageously utilized. Subsequently, the hip joint is distracted (1004) partially or fully, but preferably only enough (e.g., 1 cm) for visualization and sufficient workspace without overstressing surrounding tissues. Anterior labral resection may then be performed (1006). By inserting a dry arthroscope into the joint space, the spatial boundaries of the chondral defect are then observed and measured (1008). Measurements may be made between any two spatial points within or around the chondral defect and include without limitation: an A-P radius of curvature, an M-L radius of curvature, a defect thickness at any one or more locations, a defect length at any one or more locations, a defect width at any one or more locations, a circumference of the defect, a chord length of the defect, a radius of defect, a volume of said defect, and/or combinations thereof. After measuring (1008), the defect is removed (1010) using purpose-specific or conventional instrumentation and one or more FAI prostheses of the present invention are then installed at the prepared site (1012). Preferably, the transition surfaces between implant and cartilage are kept as smooth as possible. Next, tension on the joint may be released (1014) and the FAI implant can be secured (1016) with secondary optional fixation means (e.g., a bone screw or other means described herein). While step (1016) may be done prior to relaxing joint tension (1014), closing the joint space prior to step (1016) significantly mitigates the risk of compromising, scratching, or deforming an articular surface of the FAI implant after it is installed. Closing the joint space prior to step (1016) may also help in finding the implant's "natural home position" with respect to the intact acetabular and femoral articular cartilage. In addition to the above steps, femoral osteochondroplasty may be performed (1018) to prevent future impingement. One of ordinary skill in the art would appreciate that other steps may be introduced, and that the exact order in which the above are executed may change according to surgeon preference.

In some embodiments, it is preferred that the thickness of the lower portion (82,92,102,112) comprising the bearing surface (81,91,101,111) is minimized so as to be minimally-invasive and preserve as much intact bone and cartilage as possible. By decreasing the thickness of the implant, resection depth is kept to a minimum, thereby improving the likelihood of a successful revision to a conventional hip resurfacing or THA device.

It is preferred that the means for bio-fixation as disclosed herein does not incorporate cement; however, the devices disclosed in this specification will still function if cement is used. Cement is not preferable, since currently available cements have been linked to erosion of healthy hard and soft tissues, which could compromise revision procedures such as hip resurfacing and THA.

Subsequent arthroscopic "patching" procedures may be utilized to graft cartilage, cartilage-substitutes, bio-scaffolds, or hydrogels to the implant (80,90,100,110). The implant may have means for accepting such grafts at the periphery of the lower portion (82,92,102,112). Means for accepting grafts may comprise porous structures or suture mounts (e.g., holes). Alternatively, subsequent arthroscopic debridement may be utilized around the periphery of the implant's bearing surface (81,91,101,111) in order to form a smooth bearing transition zone. Additional material may be incorporated with the implant at the periphery of the implant for shaping and blending with adjacent cartilage. Alternatively, the implant may comprise a recess or dam for a peripheral bio-gel or hydrogel to form a smooth bearing transition zone between the implant's periphery and surrounding chondral surfaces.

Several methods may be used to implant FAI devices described herein, and instrumentation may vary in accordance to surgeon preference. FIG. 11a. illustrates one embodiment of a cutting guide (300) which may be used to remove a small portion of damaged cartilage and underlying bone in the acetabular defect region. Cutting guide (300) may be a generally L-shaped block formed of a metal or disposable polymeric material comprising a lateral block portion (302) and a distal block portion (304). Guide (300) may comprise one or more means for mounting to surrounding anatomy including, but not limited to one or more spikes (not illustrated), clamps (not illustrated), or apertures (308) configured to receive one or more holding fasteners (310). Apertures may be parallel, orthogonal or oblique to each other and configured in a pattern to provide the best stability for the guide (300) under cutting vibrations. Holding fasteners (310) may be of the headed or non-headed type including, but not limited to speed pins, threaded pins, or straight pins. One or more means for guiding a cutting tool (306) are provided on the cutting guide (300). The means may comprise, for example, one or more slots (306a, 306b, 306c) that are adapted to receive and guide an oscillating or reciprocating saw blade.

Alternatively, slots (306a, 306b, 306c) may be adapted to receive a milling or router-type cutting bit which rotates about an axis, or one or more spindles thereof. In the instance where rotating milling or router-type cutting bits are used, slots (306a, 306b, 306c) may comprise non-linear shapes to define spline curves and arcuate paths (not illustrated). If the selected FAI implant comprises one or more integral bone-engaging pegs (not shown), or receives one or more separate bone screws or pegs (372) for initial fixation and/or stability, one or more drill guides (312) may be provided to create pilot hole(s) (355) adapted to receive said one or more separate bone screws or pegs (372). Moreover, if the selected FAI implant comprises one or more tongues (108) as shown in FIGS. 8a-b, then key-type channels (208) may be made in the acetabulum by overlapping one or more drill guides (312') with a resection slot (306b) as shown in FIG. 11b. It is anticipated that drill guide (312') may comprise shapes other than what is shown in FIGS. 8a-b and FIG. 11b. For instance, instead of using a circular drill (380) in the circular drill guide (312') shown, a polygonal osteotome or broach (e.g., triangular, rectangular, octagonal) may be inserted into a correspondingly-shaped non-circular guide (312') in order to create key-type channels (208) having non-circular cross-sectional profiles. In order to improve the stability of the guide (300) during high vibrations during cutting, it is preferred that internal surfaces of the guide (300) are shaped to closely conform to the acetabular rim in the region of the defect area.

FIGS. 12a-j further illustrate method steps of using the cutting guide shown in FIG. 11a. according to some embodiments. Turning to FIG. 12a, with the cutting guide (300) pinned to the affected acetabular rim area (350) by one or more holding fasteners (310), a saw blade (360) is inserted into slot (306a) to make a controlled resection (352). A stop portion (362) on the blade (360) may be utilized to control the depth of resection potion (352) and prevent "notching" of surrounding bone and/or tissues. Stop portion (362) may comprise a lip, extension, bump, protrusion, variation in blade width, or adjustable collar which slides over the length of the blade and adjustably secures itself thereto. Alternative means for controlling resection depth may be provided, said alternative means comprising one or more laser-markings, etchings, or engravings on the blade (360) to indicate depth.

Turning to FIGS. 12b-d, similar controlled resections (354, 356, 358) are made using slots (306b, 306c) and/or edges (306d) of the cutting guide (300). Preferably, stop portions (364, 366, 368) are used to independently control the proper depth of each resection. Stop portions (362, 364, 366, 368) may be adjusted by using different blades (360) or by using a single blade having an adjustable means for depth control. Of course, controlled resections (352, 354, 356, 358) may be freehanded with a conventional blade. A drill (380) may be used to create a recess (355) for one or more separate bone screws or pegs (372). Drill (380) may also serve to create a channel (208) for an implant tongue (108) when using a cutting block such as the one shown in FIG. 11b.

Once resections are complete, bony fragment portions (350') are removed from the affected acetabular rim area (350) as shown in FIGS. 12e-12g. A correspondingly sized and shaped FAI implant (370) is then implanted in the appropriate cavity and fixed to the affected acetabular rim area (350). Fixation of the implant (370) to the acetabulum area (350) may be made by impacting, cementing, or securing with one or more bone screws or pegs (372). As shown, initial and long-term fixation and stability of the implant may be improved with the use of a non-cemented porous interface (374) and/or engagement lip (372) configured to engage an undercut resection (357) in the acetabulum. Such undercut engagements may prevent "rocking" and shifting of the implant during tightening/impacting/torquing of said one or more bone screws or pegs (372).

FIGS. 13a-d illustrate a method of using rotating cutters to mill away bone and cartilage in the defect area. In this embodiment, a cutting device (400) may be mounted to the proximal femur (F) via a means for mounting (402). Said means for mounting (402) may be pegs or barbs, or a series of apertures configured to receive mounting fasteners as described herein. Preferably, mounting of cutting device (400) is done prior to osteochondroplasty (1018) of the femur (F), so that any holes (414) or deformities (414) in the femur (F) caused by the means for mounting (402) and which might form stress-risers are removed when the protruding bone (416) causing the cam impingement is removed.

Cutting device (400) may comprise one or more cutters (404) that can be arranged to rotate independently of each other or geared to rotate together driven by a single input source. Device (400) may comprise a small sterilizable internal motor having wire leads (406). Motor (not shown) is preferably geared and adapted to effectively rotate cutters (404). Alternatively, an input drive shaft (not shown) may be provided on the device (400) which can be attached to a conventional surgical drill (not shown). The input drive shaft of the device (400) would transfer power and torque from the surgical drill to the cutters (404) thereby creating an acetabular cavity (410) configured to receive a correspondingly sized and shaped FAI implant. During the cutting process, the femur (F) may be moved throughout one or more range of motions (408) to guide the resection. By moving the cutting device (400) throughout natural ranges of motion (408) of the femur (F), location of acetabular cavity (410) and implant placement can be optimized for a particular patient.

FIGS. 14a-d illustrate a method of using rotating cutters to mill the defect area bone. Femur (F) may be distracted in direction (508) to make room for cutting device (500) to be inserted in the joint space. Cutting device (500) comprises a spoon portion (502') and a body portion (502) having one or more cutters (504) thereon. Body portion (562) is preferably integral with spoon portion (502'), but may be configured to move relative to spoon portion (502') in at least one direction (516) to allow "plunging" of the one or more cutters (504) into the defect area without moving the spoon portion (502') in the gap space. Femur (F) may be retracted in direction (512) during cutting steps.

Similar to the cutting device (400) shown in FIGS. 13a-d, cutting device (500) may comprise a small internal surgical motor having wire leads (506). Motor (not shown) is preferably geared and adapted to effectively rotate cutters (504). Alternatively, an input drive shaft (not shown) may be provided on the body portion (502) of the device (500) which can be attached to a conventional surgical drill (not shown). The input drive shaft is configured to transfer power and torque from the surgical drill to the cutters (504) and create an acetabular cavity (510) configured to receive a correspondingly sized and shaped FAI implant. During the cutting process, the femur (F) may be moved throughout one or more range of motions (514) to guide the resection. Moreover, during the cutting process, the cutting device (500) may be moved in one or more directions (518) to effectively sculpt an acetabular cavity (510).

FIGS. 15a-d illustrate a method of using a cutting block (600) mounted to a proximal femur (F) to resect away the defect area bone and cartilage (610). Femur (F) may be moved in direction (608) to make cutting block (600) flush with the outer acetabular rim. Cutting block (600) comprises one or more resection surfaces (606) which may comprise planar edge portions, captured slots, or uncaptured slots. As stated above, the resection surfaces (606) may be linear for use with an oscillating saw blade (607) or curvilinear if a router-type bit or burring tool is used (not shown).

In this embodiment, cutting device (600) may be optionally mounted to the proximal femur (F) via a means for mounting (602). Said means for mounting (602) may be pegs or barbs, or a series of apertures configured to receive mounting fasteners as previously described herein. Preferably, the mounting of cutting device (800) is done prior to osteochondroplasty (1018) of the femur (F), so that any holes (614) or deformities (614) in the femur (F) caused by the means for mounting (602) and which might form stress-risers are removed when the protruding bone (616) causing the cam impingement is removed. Mounting means (602) may also comprise a series of friction-increasing surfaces such as grooves, ridges, bumps, sharp edges, grit-blasted surfaces, etc. Alternatively, mounting means (602) may be omitted. In such instances, the cutting block is stabilized by hand and allowed to be moved relative to outer surfaces of the femur (F) which helps guide the resections.

FIGS. 16a-d illustrate a method of using a cutting guide (700) having a cutting block mounted to a spacer (702") in order to resect away the defect area bone and cartilage (710). The cutting block comprises one or more resection surfaces (706) which may comprise planar edge portions, captured slots, or uncaptured slots. As stated above, the resection surfaces (706) may be linear for use with an oscillating saw blade (707) or curvilinear if a router-type bit or burring tool is used (not shown). Spacer (702") may take many forms and shapes, but is preferably thin and spoon-shaped.

The surgeon distracts femur (F) in direction (708) to make room for spacer (702") to be inserted in the joint space. Handle (702') allows the surgeon to move spacer (702") within the joint space and position the cutting guide (700) so that resection surfaces (706) are oriented in the appropriate spatial position. Femur (F) can then be relocated by moving the femur (F) in direction (712). Relocation of the femur (F) within the joint generally stabilizes the cutting guide (700), but any additional means for mounting disclosed herein (e.g., 602) may be supplementally employed to stabilize the cutting block during resection. The cutting block is preferably integral with cutting device (700), but may be configured to move relative to spacer (702") or handle (702') in at least one direction (716) without moving the spoon portion (502') in the gap space.

FIG. 17 depicts a femoral acetabular impingement symptom-inducing device (800) for use in animal testing and FAI research. FAI symptom-inducing device (800) comprises a protrusion (804) resembling a cam-type femoral defect, and a means for mounting (802) said device (800) to a proximal portion of a healthy femur. The external shape (806) and/or size of the protrusion (804) may be varied to alter the severity of induced FAI symptoms, or to fit differently sized animal femurs. Moreover, surface roughness of the protrusion (804) may be varied to alter the severity of the induced FAI symptoms and/or to change the shape, depth, and texture of the created chondral defect.

FIG. 18 is a schematic diagram illustrating method steps for studying femoral acetabular impingement using the device shown in FIG. 17. A method (2000) for studying the affects of FAI and for researching and optimizing FAI implant geometries is disclosed. First, an FAI symptom-inducing device (800) such as the one shown in FIG. 17 is implanted (2002) on the proximal portion of one or more healthy animal femurs in order to simulate a cam-type deformity. The animal is then allowed to develop FAI symptoms (2004) for a predetermined length of time. Next, the FAI symptom-inducing implants (800) are removed (2006) from the bone (simulating femoral osteochondroplasty 1018). Observed findings are recorded (2008) and processed (2010).

The disclosed method (2000) may also be used to optimize and fine-tune FAI implant geometries. For example, the defects may be cured (2012) using one or more of the implants and surgical implantation methods described herein and the results studied over time for different implants having different shapes and sizes. FAI implant geometries and fixation means thereof may be changed so as to develop an optimal solution using in-vivo animal test data. Results from different FAI implant geometries and surgical implantation methods are recorded for comparison (2014) and regulatory compliance.

Implants of the nature disclosed may be created in a custom-made fashion, using known tomographic technologies. Preoperative CT scans, MRI scans, and X-ray templating used alone or in combination may enhance fit for an individual patient and may be used to size and locate the implant's position preoperatively. Implants may alternatively be formed intraoperatively using shaping tools which may include cutting and melting devices (for polymers and hardening polymers/gels).

Since there have been no significant studies or reportings on FAI, implants of the present invention may be studied and optimized using novel testing methods. For instance, testing methods may include the method steps of creating an animal study, inducing FAI symptoms on one side of an animal, and avoiding FAI symptoms on the opposing side of the animal by leaving the other side of the animal unaffected to serve as a control. The step of inducing FAI symptoms on one side of an animal, may include, for instance, simulating an FAI condition by forming a large protuberance on a femoral neck of a swine animal by implanting a prosthetic device. FAI treatment and implant designs may then be evaluated and optimized using this novel animal testing method. The implanted prosthetic device may be a simple button implanted on an area of the proximal femur so as to induce a "cam-type" deformity and cam impingement indication.

As various modifications could be made to the exemplary embodiments, as described above with reference to the corresponding illustrations, without departing from the scope of the invention, it is intended that all matter contained in the foregoing description and shown in the accompanying drawings shall be interpreted as illustrative rather than limiting. Thus, the breadth and scope of the present invention should not be limited by any of the above-described exemplary embodiments, but should be defined only in accordance with the following claims appended hereto.

## Claims

1. Apparatus for treating a rim deformity of an acetabulum, comprising:
a. an implant (80) having a substantially L-shaped profile in the anterior posterior coronal view, the implant comprising a first flange (82) configured to extend into the acetabulum and a second flange (84) configured substantially perpendicular to the first flange to extend over the rim of the acetabulum; and
b. a fixation element (32) configured to extend through the second flange (84) and fix the implant to chondral bone;
**characterised in that** the first flange (82) comprises a bearing surface (81) for a femoral head (10) to articulate against.

2. The apparatus of claim 1, wherein the second flange (84) has anteriorly and posteriorly extending flanges when viewed laterally.

3. The apparatus of any of the above claims, wherein a width of the first and second flanges (82, 84) is less than or equal to 2 cm and a thickness of the first and second flanges (82, 84) is between 2 and 6 mm.

4. The apparatus of any preceding claim, wherein the fixation element (32) is a screw.

5. The apparatus of any preceding claim, wherein the bearing surface (81) is for natural femoral head cartilage to articulate against.

## Patentansprüche

1. Eine Vorrichtung zum Behandeln einer Randdeformität eines Acetabulums, beinhaltend:
a. ein Implantat (80) mit einem in der Anterior-Posterior-Frontalansicht im Wesentlichen L-förmigen Profil, wobei das Implantat einen ersten Flansch (82), der konfiguriert ist, um sich in das Acetabulum zu erstrecken, und einen zweiten Flansch (84), der im Wesentlichen senkrecht zu dem ersten Flansch konfiguriert ist, um sich über den Rand des Acetabulums zu erstrecken, beinhaltet; und
b. ein Fixierungselement (32), das konfiguriert ist, um sich durch den zweiten Flansch (84) zu erstrecken und das Implantat an chondralem Knochen zu fixieren;
**dadurch gekennzeichnet, dass** der erste Flansch (82) eine Auflagefläche (81) für einen Femurkopf (10) zur Gelenkbildung dagegen beinhaltet.

2. Vorrichtung gemäß Anspruch 1, wobei der zweite Flansch (84) sich bei seitlicher Betrachtung anterior und posterior erstreckende Flansche aufweist.

3. Vorrichtung gemäß einem der vorhergehenden Ansprüche, wobei eine Breite des ersten und zweiten Flansches (82, 84) kleiner als oder gleich 2 cm ist und eine Dicke des ersten und zweiten Flansches (82, 84) zwischen 2 und 6 mm beträgt.

4. Vorrichtung gemäß einem der vorhergehenden Ansprüche, wobei das Fixierungselement (32) eine Schraube ist.

5. Vorrichtung gemäß einem der vorhergehenden Ansprüche, wobei die Auflagefläche (81) dem natürlichen Knorpel des Femurkopfes zur Gelenkbildung dagegen dient.

## Revendications

1. Appareil pour traiter une déformation du rebord d'un acétabulum, comprenant :
a. un implant (80) ayant un profil substantiellement en forme de L dans la vue coronale antérieure postérieure, l'implant comprenant une première collerette (82) configurée afin de s'étendre dans l'acétabulum et une deuxième collerette (84) configurée substantiellement perpendiculairement à la première collerette afin de s'étendre par-dessus le rebord de l'acétabulum ; et
b. un élément de fixation (32) configuré afin de s'étendre à travers la deuxième collerette (84) et fixer l'implant à l'os chondral ;
**caractérisé en ce que** la première collerette (82) comprend une surface d'appui (81) pour qu'une tête fémorale (10) s'articule contre celle-ci.

2. L'appareil de la revendication 1, dans lequel la deuxième collerette (84) possède des collerettes s'étendant antérieurement et postérieurement lorsqu'observée latéralement.

3. L'appareil de n'importe lesquelles des revendications ci-dessus, dans lequel une largeur des première et deuxième collerettes (82, 84) est inférieure ou égale à 2 cm et une épaisseur des première et deuxième collerettes (82, 84) est comprise entre 2 et 6 mm.

4. L'appareil de n'importe quelle revendication précédente, dans lequel l'élément de fixation (32) est une vis.

5. L'appareil de n'importe quelle revendication précédente, dans lequel la surface d'appui (81) est destinée à ce que du cartilage de la tête fémorale naturelle s'articule contre celle-ci.
